# EUROPEAN PATENT APPLICATION

(11) **EP 2 070 491 A2**
(43) Date of publication of application: **17.06.2009**
(21) Application number: 08253941.2
(22) Date of filing: 10.12.2008
(51) Int. Cl.: A61F 2/30, A61L 27/58

(54) **Optimum density fibrous matrix**

(30) Priority: 11.12.2007 US 954100
(71) Applicant: Advanced Technologies and Regenerative Medicine, LLC, Raynham, MA 02767 (US)
(72) Inventor: Hammer, Joseph J., Hillsborough, NJ 08844 (US); Shetty, Dhanuraj, Jersey City, NJ 07302 (US); Dhanaraj, Sridevi, Raritan, NJ 08869 (US); Wang, Ziwei, Monroe Twp., NJ 08831 (US)
(74) Representative: Tunstall, Christopher Stephen

(57) **Abstract**

An implantable biodegradable porous fibrous matrix is disclosed, the fibrous matrix being constructed of fibers arranged in a nonwoven array. The density of the nonwoven array is adjusted in the manufacturing process to obtain an optimum density of the array for tissue ingrowth. When implanted, the optimum density fibrous matrix provides for a superior biological response of the host in terms of tissue growth, especially for tissues containing glycosaminoglycans (GAGs). The optimum density fibrous matrix is therefore provided with properties useful in repair and/or regeneration of mammalian tissue.

## Description

### FIELD OF THE INVENTION

The present invention discloses biodegradable implantable tissue engineering scaffolds having an optimum density to facilitate cell infiltration, migration, and proliferation that are useful for the repair or regeneration of diseased or damaged mammalian tissue.

### BACKGROUND OF THE INVENTION

The recent emergence of tissue engineering may offer alternative approaches for the repair and regeneration of damaged or diseased tissue. Tissue engineering strategies have explored the use of biomaterials in combination with cells and/or biologically active agents to develop biological substitutes that ultimately can improve or restore tissue function. The use of colonizable and remodelable scaffolding materials has been studied extensively as tissue templates, conduits, barriers, and reservoirs. In particular, synthetic and natural materials in the form of foams, sponges, gels, hydrogels, textiles, and nonwovens have been used in vitro and in vivo to reconstruct and/or regenerate biological tissue, as well as to deliver chemotactic agents for inducing tissue growth.

Regardless of the targeted tissue and the composition of the scaffold, the scaffold must possess some fundamental characteristics to be successfully employed as a tissue engineering template. The scaffold must be biocompatible, and it must possess sufficient mechanical properties to resist tearing or crumbling while being attached to the surrounding tissue by various mechanical means, fixation devices, or adhesives. It must be highly porous to allow for the infiltration, migration, and growth of cells, such as in the polymer scaffolds described by Zwingmann, et al. (Tissue Engineering 13(9) 2335-43, 2007). The scaffold must also be able to be remodeled by the colonizing tissue, and must also be easily sterilized. Present conventional materials, either alone or in combination, are insufficient in one or more of the above criteria. Accordingly, there is a need for tissue engineering scaffolds that can resolve the potential pitfalls of conventional materials.

### SUMMARY OF THE INVENTION

Implantable, biodegradable tissue engineering scaffolds of the present invention are comprised of a porous fibrous matrix having fibers of one or more types of biodegradable materials. These fibers are processed and arranged to form a nonwoven structure having an optimum density for use as a tissue scaffold. Control of the manufacturing process allows the formation of nonwovens of specific densities, being optimized for faster cell infiltration, migration, and proliferation of chondrocytes in the nonwoven structure, wherein the chondrocytes have a maximal cellular activity. This results in faster tissue ingrowth and therefore a faster healing response. The optimum density nonwoven is therefore provided with properties useful and desirable for use in the repair and/or regeneration of mammalian tissue.

### BRIEF DESCRIPTION OF THE FIGURES

Figure 1 shows the number of bovine chondrocyte cells 168 hours after cell seeding on the various density scaffold materials.
Figure 2 shows the GAG response of bovine chondrocyte cells 168 hours after cell seeding on the various density scaffold materials.

### DETAILED DESCRIPTION OF THE INVENTION

The present invention provides biodegradable implantable tissue engineering scaffolds having a fibrous matrix that possess desirable properties for use in the repair and/or regeneration of diseased or damaged musculoskeletal tissue in mammals. In particular, the tissue engineering scaffolds of the present invention have an optimum density for faster cell infiltration, migration, and proliferation of chondrocytes in the nonwoven structure, wherein the chondrocytes have a maximal cellular activity.

It is an object of the present invention to provide a fibrous matrix that is biodegradable and resorbable by the body. It is a further object of the present invention to provide a fibrous matrix that facilitates cellular infiltration, migration, proliferation, and tissue in-growth in order for tissue to replace the resorbing fibrous matrix. It is a further object of the present invention to provide a fibrous matrix capable of providing and maintaining the structural support required for as long as is required to effect the repair and/or regeneration of the tissue, including that time period in which the fibrous matrix is being resorbed by the body. It is a further object of the present invention to provide a fibrous matrix having a density that is optimized for faster cell infiltration, migration, and proliferation of chondrocytes in the nonwoven structure, wherein the chondrocytes have a maximal cellular activity.

As described and used in this application, the terms "scaffold" and "fibrous matrix" are understood to be interchangeable and to mean a network of fibers useful for providing an implantable substrate for biological cells to attach to and proliferate. Measuring the density of the scaffold is an alternative method of characterizing the porosity thereof, and is a more convenient method that is easily utilized in the manufacturing process. Thus, the terms density and porosity are understood to be alternative terms to characterize the interstitial spaces between the fibers of the non-woven fibrous matrix that are available for cellular infiltration and ingrowth. For example, a lower density correlates with a higher porosity, and a higher density correlates with a lower porosity. The terms "degradable", "biodegradable", "resorbable" and "bioresorbable" are understood to be interchangeable and to mean a material that is biocompatible and is degraded or broken down into fragments that are either metabolized or excreted over time, but are not retained by the body. The term "GAG" is understood to have its common meaning of glycosaminoglycan, and may also include the plural glycosaminoglycans. The terms PLA and PGA are understood to have their common meanings of polylactic acid and polyglycolic acid, respectively, and are further understood to encompass various isomers and enantiomers thereof.

Biodegradable polymers that may be used to prepare fibers and fibrous matrices of the present invention include, but are not limited to, aliphatic polyesters, poly(amino acids), copoly(ether-esters), polyalkylene oxalates, polyamides, poly(iminocarbonates), polyorthoesters, polyoxaesters, polyamidoesters, poly(anhydrides), polyphosphazenes and other biopolymers. Certain of the polyoxaester copolymers may further comprise amine groups.

Biodegradable glasses that may be used to prepare fibers and fibrous matrices of the present invention include biologically active glasses comprising a silicate-containing calcium phosphate glass, e.g. BIOGLASS (TM - University of Florida, Gainesville, Fla.), or calcium phosphate glasses wherein some of the calcium ions are replaced by varying amounts of iron, sodium, magnesium, potassium, aluminum, zirconium, or other ions. This partial replacement of calcium ions is used to control the resorption time of the glass. For example, in a calcium phosphate glass with a phosphate concentration between about 50 and about 70 weight percent, substituting iron for calcium, e.g. from about 1 weight percent to about 35 weight percent iron, while keeping the phosphate level constant, will increase the time for the glass to degrade and resorb in the body.

In tissue engineering scaffolds according to the present invention, the fibrous matrix comprises an organized network of threads, yarns, nets, laces, felts, nonwovens, or combinations thereof. Preferred methods of combining the biodegradable fibrous materials, e.g. fibers, to make the fibrous matrix of the present invention are known to one skilled in the art as the "wet lay" and "dry lay" processes of forming nonwovens. These methods have been described in various references, including "Nonwoven Fabrics" (W. Albrecht, et al., eds., Wiley-VCH 2003), the contents of which are incorporated herein by reference.

The attachment, infiltration, migration, and proliferation of cells on and into a tissue engineering scaffold are separate, sequential events that are dependent on various factors and variables, one of which is the density of the scaffold. We initiated an effort to quantify the effect of the scaffold density upon the performance of the nonwoven tissue scaffolds and the cellular activity of the regenerated tissue contained therein.

The following method was used to measure and quantify the density of the nonwoven scaffold sheets. Two metal plates measuring 2.75 inches by 2.00 inches by 0.060 inches thick and having a combined weight of 82.18 grams were placed on opposite sides of an area of a nonwoven scaffold sheet to create a sandwich structure. A Mitutoyo absolute gauge (model number ID-C125EB, code number 543-452B) with a circular 1" diameter foot was used to measure the thickness of the metal-scaffold-metal sandwich. The thickness of the metal plates was then subtracted from the total to yield the thickness of the nonwoven scaffold sheet. This was repeated a minimum of four times in different areas of the sheet and the readings were then averaged to obtain a final thickness measurement. The weight of the nonwoven scaffold sheet was then measured to the nearest 0.05 gram and recorded. The length and width of the sheet were measured to the nearest millimeter. The volume of the sheet was then calculated in cubic centimeters. Finally, the density of the sheet was calculated by dividing the weight by the volume and recorded in milligrams per cubic centimeter.

We then sought to modify the density of known useful scaffold materials and compositions. Filaments of a bioresorbable copolymer comprised of 90% PGA and 10% PLA measuring approximately 20 microns in diameter were used to manufacture nonwoven sheets using a dry-lay process. The dry-lay process consisted of producing a batt of fiber using a rotary card, and the batts were then consolidated using a needle-punching process. The density of the non-woven sheet was controlled by varying the amount of starting material and varying the number of needle punches. Sheets of lower density were produced by decreasing the amount of starting material and/or by decreasing the number of needle punches. Conversely, sheets of higher density were produced by increasing the amount of starting material and/or increasing the number of needle punches. Nonwoven sheets measuring greater than 12 inches by 12 inches were produced.

A study evaluating bovine chondrocyte infiltration, proliferation, and extracellular matrix synthesis on nonwoven scaffolds made of three different densities was performed. Discs having a diameter of 5 mm were cut from the sheets to use in individual studies. Target densities of the nonwoven scaffolds were 60, 90, and 120 mg/cc. The actual densities achieved during the production process were measured using the technique described above and found to be 58.0, 85.6, and 111.2 mg/cc, respectively, thus our range of accuracy was within about 5 mg/cc. A quantity of 2.5 x 10^6 bovine chondrocyte cells were seeded onto each of the 5-mm scaffold discs, and the discs were incubated in culture medium. Chondrocyte attachment and infiltration was determined by evaluation of DNA content of the scaffolds using the CyQuant assay from molecular probes. GAG content in the scaffolds was determined by the dimethylmethylene blue assay (Sigma-Aldrich Cat # 341088).

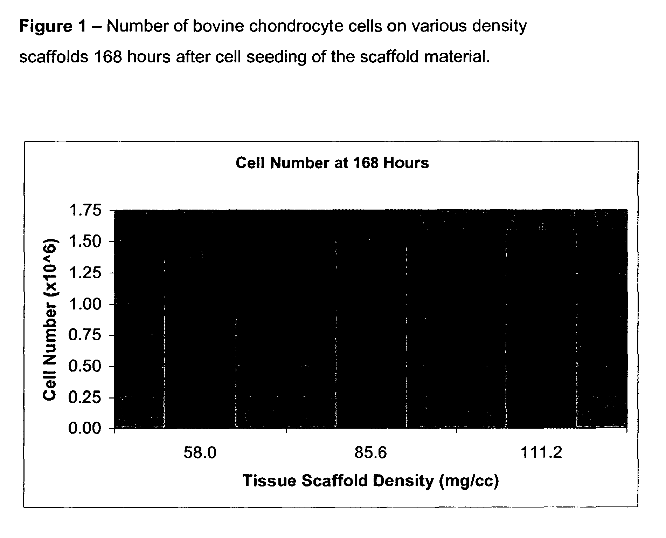

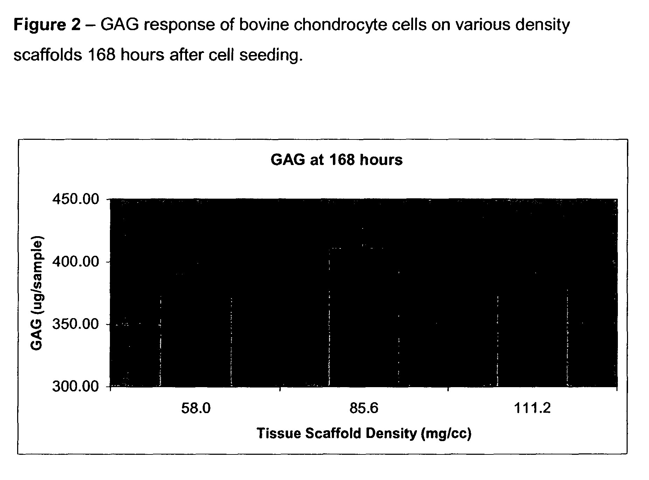

The results from chondrocyte attachment and infiltration at 168 hours following seeding showed 15-20% higher cell content in the scaffolds with the densities of 90 and 120 mg/cc as compared to the 60mg/cc density scaffolds (see figure 1). However, GAG analysis of the scaffolds after 168 hours indicate that 60 mg/cc may not be the optimal density for a tissue scaffold. The in-vitro GAG concentration of the scaffolds at 168 hours after cell seeding was measured and indicated that a maxima is reached at a scaffold density of 85.6 mg/cc (see figure 2). Thus, the optimum density for tissue engineering may not necessarily be evidenced by the number of cells present, but rather by the functional performance of the cells, such as the production of GAG by chondrocytes.

The biodegradable fibers used to prepare fibrous matrices and devices according to the present invention may be solid, or hollow, or may be of a sheath and core type of construction. Filaments may be co-extruded or could be coated to produce a sheath and core construction. For example, a co-extruded bioglass filament with a polymer sheath, or a biodegradable glass filament with a biodegradable polymer coating, or a biodegradable polymer filament with a biodegradable glass coating may form such constructs. Methods for making each construct of filament are well known to those skilled in the art. In a preferred embodiment a co-extruded construction comprising fibers with a fast-absorbing sheath surrounding a slow-absorbing core may be desirable in instances where extended support is necessary for tissue in-growth.

The nonwoven fibrous matrices of the present invention may be formed into different shapes or configurations, such as discs, circles, ovals, rectangles, squares, stars, and tubes by any convenient means known in the art, such as by punching of the nonwoven sheets with dies of appropriate shape and dimension, or by other thermal or non-thermal means of cutting or shaping the material.

In one embodiment of the invention, a continuous multifilament yarn is formed from a copolymer comprising from about 50 to about 95 weight percent PGA and from about 5 to about 50 weight percent PLA. The yarn is cut into uniform lengths between ¼" and 2". Fiber in this form is known as "staple fiber". The staple fiber comprises filaments of from about 2 to about 200 microns in diameter, preferably from about 5 to about 100 microns. The staple fiber is then used in either the dry-lay or wet-lay process to generate a nonwoven sheet of fabric that is subsequently needle punched to create a fibrous matrix of the present invention having a density of from about 58 to about 111 mg/cc.

In yet another embodiment of the invention, the porous nonwoven fibrous matrix can be chemically crosslinked or combined with hydrogels, such as alginates, hyaluronic acid, collagen gels, and poly(N-isopropylacryalmide).

In still another embodiment of the invention, the porous nonwoven fibrous matrix can be penetrated with a polymer melt or a polymer solvent solution. Such penetration provides the construct with the ability to maintain bundle coherence and retain potentially loose fibers. Biodegradable polymers that may be used to penetrate the porous nonwoven fibrous matrix are selected from the group consisting of aliphatic polyesters, poly(amino acids), copoly(ether-esters), polyalkylene oxalates, polyamides, poly(iminocarbonates), polyorthoesters, polyoxaesters, polyamidoesters, poly(anhydrides), polyphosphazenes and biopolymers.

In another embodiment of the invention, the fibrous matrix may further contain biologically active agents or factors that promote attachment, infiltration, migration, proliferation, differentiation, and/or extracellular matrix synthesis of targeted cell types. Furthermore, the bioactive agents may also comprise part of the fibrous matrix for controlled release of the bioactive agents to elicit a desired biological function. Growth factors, extracellular matrix proteins, and biologically active peptide fragments that can be used with the fibrous matrices of the current invention include, but are not limited to, members of TGF-β family, including TGF-β1, 2, and 3, bone morphogenetic proteins (BMP), fibroblast growth factors (FGF-1 and FGF-2), platelet-derived growth factors (PDGF-AA, and-BB), platelet rich plasma (PRP), vascular endothelial cell-derived growth factor (VEGF), pleiotrophin, endothelin, tenascin-C, fibronectin, vitronectin, V-CAM, I-CAM, N-CAM, selectin, cadherin, integrin, laminin, actin, myosin, collagen, microfilament, intermediate filament, antibody, elastin, fibrillin, and fragments thereof, and biological peptides containing cell-and heparin-binding domains of adhesive extracellular matrix proteins such as fibronectin and vitronectin. The biological factors may be obtained either through a commercial source, or isolated and purified from a tissue, such as from the patient to be treated.

In yet another embodiment, the three-dimensional structures of the present invention can be seeded or cultured with appropriate cell types prior to implantation for the targeted tissue. Cells which can be seeded or cultured on the fibrous matrices of the current invention include, but are not limited to, bone marrow cells, stromal cells, stem cells, embryonic stem cells, chondrocytes, intervertebral disc cells, meniscal cells, smooth muscle cells, liver cells, fibroblasts, pluripotent cells, chondrocyte progenitors, endothelial cells, macrophages, leukocytes, adipocytes, monocytes, plasma cells, mast cells, umbilical cord cells, mesenchymal stem cells, epithelial cells, myoblasts, and precursor cells derived from adipose tissue. The cells can be seeded on the scaffolds of the present invention for a short period of time, e.g. less than one day, just prior to implantation, or cultured for longer a period, e.g. greater than one day, to allow for cell proliferation and extracellular matrix synthesis within the seeded scaffold prior to implantation. In a preferred embodiment, the cell type is a chondrocyte.

The following examples are included to demonstrate preferred embodiments of the invention, and are not intended to limit the scope of the invention in any way. It will be appreciated by one skilled in the art that modifications and variations of these specific embodiments to obtain similar results fall within the spirit and scope of the invention.

### Example 1

Nonwoven sheets approximately 12 inches by 12 inches by approximately 2 millimeters thick were manufactured using filaments measuring approximately 20 microns in diameter of a copolymer comprised of 90% PGA and 10% PLA using the dry-lay nonwoven technique. The dry-lay process consisted of producing a batt of fiber using a rotary card, and the batts were then consolidated using a needle-punching process. The amount of starting material and number of needle-punches was set for a target density of 90 mg/cc, and a density of 85.6 mg/cc was actually obtained. Discs measuring 5-mm in diameter were punched out from the sheet and sterilized by ethylene oxide gas sterilization. The discs were then seeded with 2.5 x 10^6 bovine chondrocyte cells and placed in chondrocyte growth media comprised DMEM-High glucose, supplemented with 10% fetal calf serum [FCS], 10 mM HEPES, 0.1 mM nonessential amino acids, 20 µg/ml of L-proline, 50 µg/ml ascorbic acid, 100 u/ml penicillin, 100 µg/ml of streptomycin and 0.25 µg/ml of amphotericin B, and then incubated at 37 degrees Celsius.

After 168 hours the scaffold discs were subject to analysis for chondrocyte attachment as determined by evaluation of the DNA content using the CyQuant™ assay from Molecular Probes™, and the GAG content in the scaffolds was determined by dimethylmethylene blue assay (Sigma-Aldrich Cat # 341088). The number of cells attached was found to be 1.5 x 10^6 and the GAG content was 410 ug per scaffold.

### Example 2

Nonwoven sheets were prepared as in example 1, except that a target density of 60 mg/cc was used and an actual density of 58.0 mg/cc was obtained. Scaffold discs of 5-mm diameter were also prepared as in example 1 and subjected to cell seeding, incubation, and analysis as in example 1. The number of cells attached was found to be 1.3 x 10^6 and the GAG content was 388 ug per scaffold.

### Example 3

Nonwoven sheets were prepared as in example 1, except that a target density of 120 mg/cc was used and an actual density of 111.2 mg/cc was obtained. Scaffold discs of 5-mm diameter were also prepared as in example 1 and subjected to cell seeding, incubation, and analysis as in example 1. The number of cells attached was found to be 1.58 x 10^6 and the GAG content was 382 ug per scaffold.

### Example 4

A nonwoven sheet is prepared from a fiber comprised of a fast degrading outer sheath comprised of a 50/50 copolymer of PGA/PLA, and an inner core comprised of a slower degrading fiber of 90/10 PGA/PLA copolymer. The amount of starting material and needle punching is adjusted to achieve a nonwoven sheet having a density of about 90 mg/cc. The nonwoven sheet thus formed is placed in a sterile barrier package and sterilized by ethylene oxide gas. At the time of use to treat a patient having a musculoskeletal defect, the nonwoven sheet is removed from the package, cut into the desired size and shape to complement the defect site to be treated, and the scaffold is then seeded with chondrocyte cells previously obtained and cultured from the patient to be treated. A bioactive agent is added to the scaffold to enhance the therapy, and the scaffold is then fixed in place using resorbable sutures, thereby providing for repair and regeneration of the tissue defect site.

### Example 5

A nonwoven sheet is prepared from a fiber comprised of a biodegradable glass. The amount of starting material and needle punching is adjusted to achieve a density of about 90 mg/cc. The nonwoven sheet thus formed is placed in a sterile barrier package and sterilized by ethylene oxide gas. At the time of use to treat a patient having a musculoskeletal defect the nonwoven sheet is removed from the package, cut into the desired size and shape to complement the defect site to be treated, and the scaffold is then seeded with chondrocyte cells previously obtained and cultured from the patient to be treated. A bone morphogenetic protein is added to one side of the scaffold to enhance the attachment to the bone surface of the patient and the scaffold is then fixed in place using resorbable sutures, thereby providing for repair and regeneration of the tissue defect site.

## Claims

1. An implantable biodegradable porous nonwoven fibrous matrix device comprising fibers, and further having a density between 58 and 111 mg/cc.

2. The device of claim 1 wherein the density is about 85 mg/cc .

3. The device in any preceding claim wherein the fibers are selected from the group consisting of synthetic polymers, natural polymers, and biodegradable glasses.

4. The device in claim 3 wherein the synthetic polymer fibers are comprised of one or more copolymers.

5. The device of any preceding claim wherein the fibers are further comprised of an outer sheath of a faster degrading material and an inner core of a slower degrading material.

6. The device of any preceding claim wherein the fibers have a diameter from about 2 microns to about 200 microns.

7. The device of any preceding claim wherein the fibrous matrix is chemically crosslinked.

8. The device of any preceding claim wherein the fibrous matrix further comprises a biological agent.

9. The device of claim 8 wherein the biological agent is selected from the group consisting of biological cells, TGF-β1, TGF-β2, TGF-β3, bone morphogenetic protein, fibroblast growth factor, platelet-derived growth factor, platelet rich plasma,vascular endothelial cell-derived growth factor, pleiotrophin, endothelin, tenascin-C, fibronectin, vitronectin, V-CAM, I-CAM, N-CAM, selectin, cadherin, integrin, laminin, actin, myosin, collagen, microfilament, intermediate filament, antibody, elastin, fibrillin, fibronectin, and vitronectin.

10. The device of claim 9 wherein the biological agent is chondrocyte cells.

11. The device of any preceding claim wherein the fibrous matrix is penetrated with a polymer solution.

12. The device of any preceding claim wherein the device is sterile.

13. A method of treating a mammal in need of tissue repair comprising the steps of:
a. providing an implantable biodegradable porous nonwoven fibrous matrix having a density of from about 58 to about 111 mg/cc,
b. implanting said fibrous matrix into a defect site of said mammal.

14. The method of claim 13 further comprising the step of seeding said fibrous matrix with chondrocyte cells.

15. The method of claim 14 wherein said chondrocyte cells are cultured in said matrix before implantation in said mammal.
